# FASCICULE DE BREVET EUROPEEN

(11) **EP 1 456 155 B1**
(45) Date de publication et mention de la délivrance du brevet: **19.09.2007**
(21) Numéro de dépôt: 02795367.8
(22) Date de dépôt: 05.11.2002
(51) Int. Cl.: C07C 15/107, C07C 2/66

(54) **PROCEDE DE PRODUCTION DE PHENYLALCANES UTILISANT UNE COMBINAISON DE DEUX CATALYSEURS**
VERFAHREN ZUR HERSTELLUNG VON PHENYLALKANEN UNTER ANWENDUNG EINER KOMBINATION VON ZWEI KATALYSATOREN
METHOD FOR PRODUCING PHENYLALKANES USING A COMBINATION OF TWO CATALYSTS

(30) Priorité: 15.11.2001 FR 0114885
(43) Date de publication de la demande: 15.09.2004
(73) Titulaire: Institut Français du Pétrole, 92852 Rueil-Malmaison Cedex (FR)
(72) Inventeur: JOLY, Jean-François, F-69006 Lyon (FR); BRIOT, Patrick, F-38260 Pommier de Beaurepaire (FR)
(86) Numéro de dépôt international: PCT/FR2002/003793
(87) Numéro de publication internationale: WO 2003/042137

(56) Documents cités:
- WO-A-97/47573

## Description

La présente invention se rapporte au domaine des procédés de production de phénylalcanes par alkylation du benzène au moyen d'au moins une mono-oléfine, le plus souvent linéaire, et comprenant en général de 9 à 16 atomes de carbone par molécule. La réaction d'alkylation est effectuée en présence d'au moins deux catalyseurs différents, utilisés dans au moins deux zones réactionnelles distinctes. La présente invention permet notamment d'ajuster et de moduler selon les besoins la quantité d'isomère 2-phénylalcane produite tout en diminuant les proportions de composés lourds polyalkylés issus de la réaction d'alkylation. Selon un exemple d'application non limitatif, les phénylalcanes obtenus selon l'invention constituent des précurseurs pour la formulation, par exemple après sulfonation, de détergents et en particulier de certains détergents biodégradables.

Actuellement, les bases pour détergents biodégradables font largement appel aux alkylbenzènes linéaires. La production de ce type de composés est en croissance régulière. Une des propriétés principales recherchée pour ces composés, après une étape de sulfonation, est, outre leur pouvoir détergent, leur biodégradabilité. Pour assurer une biodégradabilité maximale, le groupement alkyle doit être linéaire et long et la distance entre le groupe sulfonate et le carbone terminal de la chaîne linéaire doit être maximale. Ainsi, les agents d'alkylation du benzène les plus intéressants sont constitués par les oléfines linéaires en C₉-C₁₆, et de préférence en C₁₀-C₁₄.

Les alkylbenzènes linéaires généralement obtenus par alkylation du benzène au moyen d'oléfine(s) linéaire(s) sont préparés le plus souvent par deux procédés connus. Le premier procédé, décrit par exemple dans l'encyclopédie Ullmann (5 ^{ième} volume A 25, page 766) utilise, lors de l'étape d'alkylation du benzène, de l'acide fluorhydrique comme catalyseur acide. Le second procédé, décrit par exemple dans l'encyclopédie Ullmann (5 ^{ième} volume A 25, page 766), utilise un catalyseur de type Friedel et Crafts, généralement à base de AlCl₃. Ces deux procédés conduisent à la formation des isomères 2-, 3-, 4-, 5- et 6-phénylalcanes. Le principal inconvénient de ces procédés est lié à des contraintes d'environnement. Le premier procédé, basé sur l'utilisation d'acide fluorhydrique, pose des problèmes de sécurité sévères d'une part et de retraitement de déchets d'autre part. Le second procédé, basé sur l'utilisation d'un catalyseur de type Friedel et Crafts, pose le problème des rejets issus de l'utilisation d'un tel type de catalyseur. En effet, il est nécessaire dans ce cas de neutraliser les effluents par une solution basique en sortie de réacteur. De plus, la séparation du catalyseur des produits de la réaction est nécessaire et difficile à mettre en oeuvre pour les deux procédés.
Ces diverses contraintes expliquent l'intérêt qu'il y a à mettre au point un procédé d'alkylation du benzène par les oléfines et plus particulièrement par des oléfines linéaires en présence d'un catalyseur solide.

L'art antérieur fait essentiellement état de l'utilisation de catalyseurs possédant des propriétés de sélectivité géométrique et conduisant à une sélectivité améliorée en 2- et 3-phénylalcanes. Lesdits catalyseurs présentant des propriétés de sélectivité géométrique sont généralement constitués de composés zéolithiques tels que définis dans la classification *"Atlas of Zeolite Structure Types"* (W. M Meier, D. H. Olson and Ch. Baerlocher, 4th revised edition, 1996, Elsevier) auquel se réfère également la présente demande. Ainsi le brevet US-A-4,301,317 décrit toute une série de zéolithes parmi lesquelles la cancrinite, la gmélinite, la mordénite, l'offrétite et la ZSM-12. Dans le brevet FR-B-2,697,246, déposé par la Demanderesse, il est montré que l'on peut utiliser des catalyseurs à base de zéolithe Y désaluminée. Le brevet EP-B1-160 144 décrit l'utilisation de zéolithes partiellement cristallisées, notamment de zéolithes Y dont la cristallinité varie de 30 à 80% tandis que le brevet US-A-5,036,033 enseigne l'utilisation de zéolithes Y riches en cations ammonium. Le brevet US-A-4,301,316 montre que la nature du catalyseur influe directement sur la composition des différents isomères phénylalcanes produits. Après réaction du 1-dodécène sur le benzène en présence de divers catalyseurs, les sélectivités en isomères phénylalcanes données dans l'art antérieur ont été reportées dans le tableau 1. Il apparaît clairement que pour un catalyseur de structure donnée, la proportion d'isomère 2-phénylalcane résulte essentiellement des caractéristiques intrinsèques du catalyseur. L'emploi du catalyseur HF conduit à une proportion de 20% de 2-phénylalcane, tandis que celui de la ZSM-12 conduit à une proportion de 92% de 2-phénylalcane.

**Tableau 1**

| Catalyseur | 2-ϕ (%) | 3-ϕ (%) | 4-ϕ(%) | 5-ϕ(%) | 6-ϕ(%) |
|---|---|---|---|---|---|
| ZSM-12 | 92 | 8 | 0 | 0 | 0 |
| Mordénite | 85 | 15 | 0 | 0 | 0 |
| Offrétite | 79 | 14 | 5 | 1 | 1 |
| ZSM-4 | 57 | 25 | 8 | 5 | 5 |
| Beta | 57 | 18 | 10 | 7 | 8 |
| Linde L | 40 | 18 | 16 | 15 | 11 |
| ZSM-38 | 37 | 19 | 13 | 14 | 16 |
| ZSM-20 | 51 | 21 | 11 | 9 | 8 |
| REY | 25 | 20 | 18 | 19 | 18 |
| HF | 20 | 17 | 16 | 23 | 24 |
| AlCl3 | 32 | 22 | 16 | 15 | 15 |

Le brevet US-A-6,133,492 divulgue un procédé de production d'alkylbenzènes linéaires conduisant à une sélectivité élevée en 2-phénylalcane. Ce procédé antérieur utilise deux réacteurs en série : le premier réacteur contient un catalyseur à base de zéolithe mordénite contenant du fluor et le second réacteur contient un second catalyseur d'alkylation dont la sélectivité en 2-phénylalcane est inférieure à celle du catalyseur à base de mordénite fluorée. Le second catalyseur d'alkylation est de préférence choisi dans le groupe constitué par les silice-alumine fluorées, les argiles contenant du fluor et le chlorure d'aluminium. Ce procédé antérieur, même s'il conduit à une sélectivité élevée en 2-phénylalcane, ne donne pas satisfaction quant à la répartition des produits obtenus en sortie du second réacteur: les composés dialkylés et lourds présentant plusieurs chaînes linéaires sur le noyau benzénique sont en effet produits en quantité non négligeable, ce qui limite la proportion de composés monoalkylés pourtant recherchés dans la composition finale.
Aussi la présente invention se propose de fournir un procédé d'alkylation de composés aromatiques, préférentiellement du benzène, au moyen d'oléfine(s) linéaire(s) comprenant de 9 à 16 atomes de carbone par molécule, plus particulièrement de 10 à 14 atomes de carbone par molécule, permettant non seulement d'ajuster la sélectivité en 2-phénylalcane à un niveau désiré mais également d'augmenter la proportion de composés monoalkylés produits, c'est-à-dire ne contenant qu'une seule chaîne linéaire sur le noyau benzénique, et menant en conséquence à une production de composés di-alkylés et de composés lourds plus faible.

Le procédé selon l'invention met en oeuvre au moins deux zones réactionnelles distinctes contenant chacune au moins un catalyseur dont les caractéristiques sont précisées ci-après. Le procédé selon la présente invention utilise ainsi au moins deux catalyseurs, les catalyseurs employés dans chacune desdites zones réactionnelles étant différents l'un de l'autre et la sélectivité en produits monoalkylés du catalyseur contenu dans la première zone réactionnelle étant inférieure à la sélectivité du catalyseur contenu dans la deuxième zone réactionnelle, située en aval de la première dans le sens de circulation des fluides.
Avantageusement, au moins l'un desdits catalyseurs contenus dans lesdites zones réactionnelles distinctes comprend au moins une zéolithe. Des zéolithes particulièrement intéressantes pour la mise en oeuvre du procédé selon l'invention sont notamment choisies dans le groupe constitué par les zéolithes appartenant aux types structuraux FAU, MOR, MTW, OFF, MAZ, BEA et EUO. La zéolithe Y appartenant au type structural FAU, la mordénite appartenant au type structural MOR, la zéolithe ZSM-12 appartenant au type structural MTW, l'offrétite appartenant au type structural OFF, la zéolithe ZSM-4 appartenant au type structural MAZ et la zéolithe beta appartenant au type structural BEA sont particulièrement préférées. La zéolithe REY qui est une zéolithe de type faujasite, fortement acide, peut également être utilisée.
Selon un mode particulier de réalisation de l'invention, on utilise dans l'une des zones réactionnelles un catalyseur contenant une zéolithe Y. Il va de soi que selon la nature et les caractéristiques, notamment en terme de sélectivité en composés monoalkylés, de l'autre catalyseur utilisé en combinaison avec le catalyseur à base de zéolithe Y et également selon la proportion en 2-phénylalcane désirée, le catalyseur à base de zéolithe Y occupera soit la première zone réactionnelle soit la seconde zone réactionnelle, située en aval de la première dans le sens de la circulation des fluides. La zéolithe Y présente dans l'un des catalyseurs utilisés dans l'une quelconque desdites zones réactionnelles est avantageusement une zéolithe Y désaluminée, de rapport atomique Si/Al global supérieur à 4, de préférence compris entre 8 et 70 et de manière encore plus préférée compris entre 15 et 25, et ne contenant pas d'espèces aluminiques externes au réseau cristallin. Les zéolithes Y désaluminées et leur préparation sont connues : on pourra par exemple se référer à l'enseignement du brevet US-A-4,738,940. La zéolithe Y désaluminée est employée en mélange avec un liant ou une matrice généralement choisi(e) dans le groupe formé par les argiles, les alumines, la silice, la magnésie, la zircone, l'oxyde de titane, l'oxyde de bore et toute combinaison d'au moins deux de ces oxydes comme la silice-alumine ou la silice-magnésie. Toutes les méthodes connues d'agglomération et de mise en forme sont applicables, telles que, par exemple, l'extrusion, le pastillage et la coagulation en gouttes. Ainsi, selon le mode particulier de réalisation de l'invention consistant à utiliser un catalyseur à base d'une zéolithe Y désaluminée, ledit catalyseur contient généralement de 1 à 100 %, de préférence de 20 à 98 % et de manière encore plus préférée de 40 à 98 % en poids de ladite zéolithe Y désaluminée et de 0 à 99 %, de préférence de 2 à 80 %, et de manière plus préférée de 2 à 60 % en poids d'une matrice ou d'un liant. Selon ce mode particulier de réalisation de l'invention, la zéolithe Y utilisée peut également être une zéolithe acide HY caractérisée par différentes spécifications et notamment un rapport atomique Si/Al global supérieur à 4, de préférence compris entre 8 et 70, et de manière encore plus préférée compris entre 15 et 25, une teneur en sodium inférieure à 0,25 % en poids, un paramètre cristallin de la maille élémentaire inférieur à 24,55.10⁻¹⁰ m et, de manière préférée, compris entre 24,21.10⁻¹⁰ m et 24,39.10⁻¹⁰ m, une surface spécifique déterminée par la méthode B.E.T. supérieure à environ 300 m²/g, de préférence supérieure à environ 450 m²/g et une capacité d'adsorption de vapeur d'eau à 25°C, pour une pression partielle de 3,46 mbar (millibar), supérieure à environ 0,5 % et de préférence, supérieure à environ 3 %.
La proportion d'espèces aluminiques extra-réseau de la zéolithe Y désaluminée étant très faible voire même nulle, il n'est pas détecté de signal attribuable à de telles espèces, ni par résonance magnétique nucléaire de ²⁷Al en utilisant la technique de la rotation à l'angle magique, ni par spectroscopie infrarouge dans la région des groupes hydroxyles. De manière plus quantitative, le rapport de l'intensité des signaux correspondant aux espèces aluminiques hors charpente (extra-réseau) sur l'intensité des signaux correspondant aux espèces aluminiques de charpente est inférieur à 0,05, quelle que soit la technique de caractérisation utilisée.
Le rapport atomique Si/Al global est généralement mesuré par analyse chimique. Quand la quantité d'aluminium est faible, par exemple inférieure à 2 %, il est opportun d'utiliser une méthode de dosage par spectrométrie d'adsorption atomique.
Le paramètre de maille peut être calculé à partir du diagramme de diffraction des rayons X, selon la méthode décrite dans la fiche ASTM D 3942-80. Pour effectuer ce calcul, il convient que la cristallinité du produit soit suffisante.
La surface spécifique est par exemple déterminée par mesure de l'isotherme d'adsorption d'azote à la température de l'azote liquide et calculée selon la méthode classique B.E.T. Les échantillons sont prétraités, avant la mesure, à 500°C sous balayage d'azote sec.
Les pourcentages de reprise en eau (ou capacité d'adsorption de vapeur d'eau) sont par exemple déterminées à l'aide d'un appareillage classique de gravimétrie. L'échantillon est prétraité à 400°C sous vide primaire, puis porté à une température stable de 25°C. On admet ensuite une pression d'eau de 3,46 mbar, ce qui correspond à un rapport P/Po d'environ 0,10 (rapport entre la pression partielle d'eau admise dans l'appareil et la pression de vapeur saturante de l'eau à la température de 25°C).
Les zéolithes Y sont généralement fabriquées à partir d'une zéolithe NaY par une combinaison appropriée de deux traitements de base : a) un traitement hydrothermique qui associe température et pression partielle de vapeur d'eau, et b) un traitement acide réalisé de préférence à l'aide d'un acide minéral fort et concentré. Généralement la zéolithe NaY à partir de laquelle on prépare la zéolithe Y utilisée pour la réalisation de l'invention possède un rapport atomique Si/Al global compris entre environ 1,8 et 3,5 ; il convient au préalable d'en abaisser la teneur pondérale en sodium à moins de 3 % et de préférence à moins de 2,5 %. L'abaissement de la teneur en sodium peut s'effectuer par échanges ioniques de la zéolithe NaY dans des solutions de sel d'ammonium (nitrate, sulfate, oxalate etc..) de concentration en ammonium comprise entre 0,01 et 10 N, à une température comprise entre 10 et 180°C (échange sous pression autogène éventuellement), pendant une durée supérieure à 10 minutes environ. La zéolithe NaY possède en outre généralement une surface spécifique comprise entre environ 750 et 950 m²/g.

Selon un autre mode particulier de réalisation de l'invention, on utilise dans l'une des zones réactionnelles un catalyseur contenant une zéolithe mordénite. Il va de soi que selon la nature et les caractéristiques, notamment en terme de sélectivité en composés monoalkylés, de l'autre catalyseur utilisé en combinaison avec le catalyseur à base de zéolithe mordénite et également selon la proportion en 2-phénylalcane désirée, le catalyseur à base de zéolithe mordénite occupera soit la première zone réactionnelle soit la seconde zone réactionnelle, située en aval de la première dans le sens de la circulation des fluides. La zéolithe mordénite présente dans l'un des catalyseurs utilisés dans l'une quelconque desdites zones réactionnelles est avantageusement une zéolithe mordénite non fluorée. Elle présente en général un rapport atomique Si/Al global compris entre 6 et 100, de préférence entre 15 et 60 et de manière encore plus préférée entre 20 et 50, une teneur pondérale en sodium inférieure à 1000 ppm, de préférence inférieure à 500 ppm, un volume microporeux, mesuré par adsorption d'azote à 77 K sous une pression partielle d'azote de 0,19, supérieur à 0,160 cm³ (liquide)/g, de préférence supérieur à 0,180 cm³ (liquide)/g. La préparation du catalyseur à base d'une zéolithe de type structural MOR, de préférence la zéolithe mordénite, consiste, dans un premier temps, à éliminer la majeure partie des cations sodium présent dans ladite zéolithe et à les remplacer par des protons puis, dans un second temps, à optimiser les rapports Si/Al globaux et de charpente. Pour éliminer la majeure partie des cations sodium, on peut par exemple procéder à une ou plusieurs série(s) d'échanges dans des solutions de sel d'ammonium (chlorure, nitrate ou sulfate d'ammonium par exemple) dans une solution de normalité comprise entre 0,01 et 15N ou dans des solutions d'acides divers (HCl, H₂SO₄, HNO₃ de normalité peu élevée), à une température comprise entre 10 et 180°C. Ledit échange pourra éventuellement être réalisé sous une pression autogène pendant une durée supérieure à 10 minutes environ.
Pour obtenir les rapports atomiques Si/Al globaux et de charpente désirés, on peut recourir aux techniques de désalumination connues de l'homme du métier, telles que par exemple l'attaque acide directe de la forme sodique de la mordénite (NaMOR) échangée partiellement ou non par les ions H⁺ ou NH₄⁺ (forme HMOR ou NH₄MOR respectivement), la calcination en présence éventuellement de vapeur d'eau de la forme HMOR ou NH₄MOR suivie, de préférence, d'un traitement chimique du type attaque acide. L'attaque acide consiste en au moins un traitement dans des solutions d'acide de nature diverse (HCl, HNO₃, H₂SO₄, HF etc), à des températures comprises entre 50°C et 150°C (attaque sous pression autogène éventuellement). Les concentrations en acide sont comprises entre 0,5 et 15N et, de préférence, entre 5 et 12N. Les rapports volume de solution sur poids de solide sec sont compris entre 3 et 20 cm³/g et, de manière avantageuse, entre 3 et 7 cm³/g. La durée du traitement est d'au moins 10 minutes. Pour atteindre les spécifications désirées, on peut avoir recours à un nombre limité d'attaques acides réalisées dans des conditions sévères ou à un nombre plus important d'attaques réalisées dans des conditions modérées. Le traitement acide direct sur la mordénite peut également permettre d'éliminer la majeure partie des cations sodium et donc éviter l'étape préalable d'échange cationique. Le traitement thermique en présence de vapeur d'eau consiste habituellement en une calcination réalisée à une température supérieure à 350°C et, de préférence, supérieure à 500°C, pendant une durée d'au moins 10 minutes, sous une atmosphère contenant au moins 1 % de vapeur d'eau, de préférence au moins 10 % de vapeur d'eau. L'attaque acide qui suit éventuellement la calcination est réalisée dans les mêmes conditions que l'attaque acide décrite précédemment. La mordénite ainsi préparée peut être utilisée, selon la présente invention, seule ou en mélange avec un liant ou une matrice généralement choisi(e) dans le groupe formé par les argiles, les alumines, la silice, la magnésie, la zircone, l'oxyde de titane, l'oxyde de bore et toute combinaison d'au moins deux des composés précités préférentiellement la silice-alumine ou la silice-magnésie. Toutes les méthodes connues d'agglomération et de mise en forme sont applicables, telles que par exemple l'extrusion, le pastillage, la coagulation en gouttes et le séchage par atomisation. Ainsi, selon le mode particulier de réalisation de l'invention consistant à utiliser un catalyseur à base d'une mordénite, ledit catalyseur contient généralement de 1 à 100 %, de préférence de 20 à 98 % et de manière encore plus préférée de 40 à 98 % en poids de ladite mordénite et de 0 à 99% de préférence de 2 à 80 % et de manière très préférée de 2 à 60 % en poids d'une matrice ou d'un liant.

Pour la mise en oeuvre du procédé selon l'invéntion, il peut également être très avantageux d'employer dans chacune desdites zones réactionnelles distinctes, au moins un catalyseur contenant au moins une zéolithe (catalyseur zéolithique). Conformément à l'invention, les zéolithes présentes dans chacun des catalyseurs zéolithiques diffèrent l'une de l'autre par leur type structural et/ou par la composition chimique de leur charpente cristalline. Par exemple, on peut utiliser deux catalyseurs zéolithiques contenant chacun une zéolithe de type structural différent. On peut également utiliser deux catalyseurs zéolithiques contenant chacun une zéolithe de type structural identique mais ayant une composition chimique de la charpente cristalline différente, c'est-à-dire présentant par exemple un rapport Si/Al différent, de telle façon que chacun des catalyseurs zéolithiques présente une sélectivité différente pour les composés monoalkylés, par exemple après un traitement de maturation différent. Toute combinaison de deux catalyseurs zéolithiques différant par le type structural et/ou par la composition chimique de la charpente cristalline des zéolithes employées et dans laquelle la sélectivité en composés monoalkylés du catalyseur utilisé dans la première zone est inférieure à celle du catalyseur utilisé dans la seconde zone peut être envisagée pour la mise en oeuvre du procédé de l'invention en vue d'obtenir la sélectivité désirée en 2-phénylalcane et une sélectivité optimale en composés monoalkylés. Selon ce mode de réalisation de l'invention dans lequel au moins deux catalyseurs zéolithiques sont combinés, une combinaison particulièrement préférée est l'association d'un catalyseur à base d'une zéolithe de type structural FAU, notamment la zéolithe Y, et d'un catalyseur à base d'une zéolithe de type structural MOR, notamment la zéolithe mordénite, la zéolithe de type structural FAU étant contenue dans le premier catalyseur i.e. dans le catalyseur présent dans la première zone réactionnelle et la zéolithe de type structural MOR étant contenue dans le second catalyseur i.e. dans la deuxième zone réactionnelle située en aval de la première dans le sens de la circulation des fluides. Les zéolithes Y et MOR contenues dans chacun des catalyseurs zéolithiques présentent des propriétés physico-chimiques similaires à celles décrites précédemment. Au moyen de cette combinaison des zéolithes Y et mordénite, il est possible de régler la proportion de 2-phénylalcane en sortie du procédé entre environ 25% en poids et environ 85 % en poids.
Le procédé objet de la présente demande peut être réalisé dans un seul réacteur, généralement un réacteur en lit fixe, au sein duquel sont présentes les zones réactionnelles contenant les catalyseurs, ou peut également être réalisé dans des réacteurs en série, chacun d'eux contenant une seule zone catalytique contenant un type de catalyseur. Cette seconde configuration sera le plus souvent préférée car elle permet de manière générale de mieux prendre en compte les propriétés et les conditions réactionnelles optimales associées à chaque catalyseur, par exemple en ajustant de manière indépendante les températures des deux réacteurs.
Selon un mode de mise en oeuvre du procédé selon l'invention, la(les) oléfine(s) est (sont) mélangée(s) avec le(les) composé(s) aromatique(s) en amont de la première zone réactionnelle.
Selon un autre mode préféré de mise en oeuvre du procédé selon l'invention, une première fraction de la(des) oléfine(s) est mélangée avec le(les) composé(s) aromatique(s) en amont de la première zone catalytique et une deuxième fraction de la(des) oléfine(s) est mélangée avec au moins une partie des effluents issus de la première zone réactionnelle. Préférentiellement, la quantité d'oléfine(s) contenue dans ladite première fraction est telle que sensiblement la totalité de la (desdites) oléfine(s) est consommée dans la première zone réactionnelle.
En général, la réaction d'alkylation est suivie d'au moins une étape de séparation des réactifs en excès. Elle est également avantageusement suivie d'au moins une étape de séparation des composés monoalkylés issus de la réaction.
Selon une mode d'application non limitatif du procédé selon l'invention, on fait réagir du benzène avec une charge renfermant au moins une oléfine linéaire comprenant de 9 à 16 atomes de carbone par molécule, de préférence de 10 à 14 atomes de carbone par molécule, la charge pouvant contenir des paraffines. La totalité du benzène peut être introduite à l'entrée de la première zone réactionnelle contenant le premier catalyseur, le mélange contenant les oléfines linéaires pouvant être dans sa totalité introduit à l'entrée de la première zone, ou préférentiellement fractionné en au moins deux parties, l'une d'elle étant introduite à l'entrée de la première zone de catalyseur, une autre étant introduite à l'entrée de la seconde zone, située en aval de la première dans le sens de circulation des fluides. Dans le cas ou un seul réacteur comprenant plusieurs zones réactionnelles est utilisé, on fera par exemple une injection latérale dans le réacteur dans une zone située entre deux zones réactionnelles.

A la sortie du ou des réacteurs, donc de la zone contenant le second catalyseur, on fractionne en général le produit obtenu de manière à recueillir séparément une première fraction renfermant du benzène non converti, une deuxième fraction renfermant au moins une oléfine linéaire C₉-C₁₆ (de préférence C₁₀-C₁₄) non convertie ainsi que les paraffines éventuellement initialement présentes dans la charge, une troisième fraction renfermant des 2-, 3-, 4-, 5- et 6-phénylalcanes et une quatrième fraction renfermant au moins un poly-alkylbenzène (ou fraction poly-alkylbenzène), celle-ci pouvant être éventuellement, au moins en partie, recyclée vers l'une des deux zones réactionnelles où elle réagit avec du benzène au contact du catalyseur présent dans la zone catalytique concernée, afin d'être au moins en partie transalkylée (réaction de transalkylation), et on recueille un mélange de 2-, 3-, 4-, 5- et 6-phénylalcanes.
De même et de manière préférée, la deuxième fraction renfermant au moins une oléfine linéaire C₉-C₁₆ (habituellement C₁₀-C₁₄) non convertie est au moins en partie recyclée vers l'une des deux zones réactionnelles.
Les conditions opératoires appliquées aux deux zones réactionnelles sont bien entendues choisies par l'homme de l'art en fonction de la structure du catalyseur, la pression totale étant cependant sensiblement la même (aux pertes de charges près) pour les deux zones réactionnelles. Les deux zones réactionnelles sont opérées à une température habituellement inférieure à 400 °C, de préférence inférieure à 300°C est de manière encore plus préférée inférieure à 250 °C et sous une pression de 1 à 10 MPa, avec un débit d'hydrocarbures liquides (vitesse spatiale) d'environ 0,5 à 50 volumes par volume de catalyseur et par heure et un rapport molaire benzène/(oléfine linéaires C₉-C₁₆) compris entre 1 et 20. Des températures différentes peuvent bien entendu être appliquées aux deux zones réactionnelles, dans le cas où deux réacteurs séparés sont utilisés.

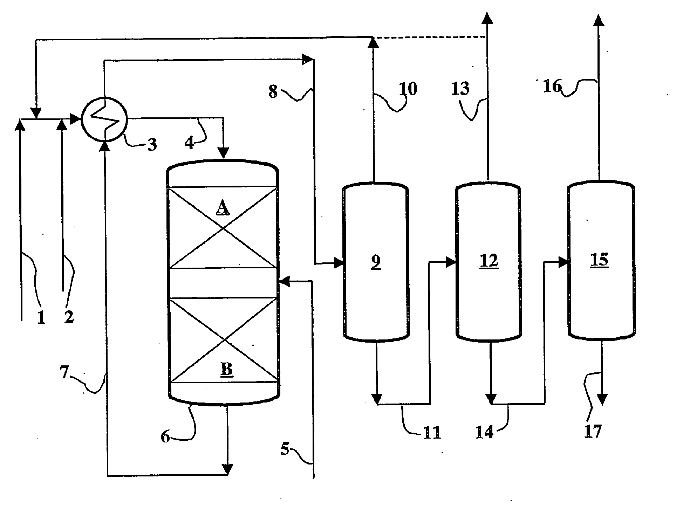

Un mode particulier de réalisation d'un dispositif permettant la mise en oeuvre du procédé de l'invention est décrit en relation avec la figure annexée. Il n'est en rien limitatif.

Du benzène frais arrivant par la conduite 1 est mélangé avec du benzène provenant de la tête d'une première colonne de fractionnement 9 (conduite 10). Cette charge constituée de benzène est mélangée avec un flux comprenant des oléfines linéaires C₉-C₁₆, de préférence des oléfines linéaires C₁₀-C₁₄, et des paraffines majoritairement C₁₀-C₁₄ (conduite 2). Le mélange global obtenu constitue la charge d'un réacteur d'alkylation 6. Ladite charge traverse préalablement un échangeur de chaleur 3 où elle est préchauffée par échange de chaleur indirect avec un effluent issu du réacteur d'alkylation 6. La charge est ensuite envoyée, après son séjour dans l'échangeur de chaleur 3, dans le réacteur d'alkylation 6 par la conduite 4. Le réacteur d'alkylation 6 est caractérisé en ce qu'il comprend deux lits réactionnels distincts A et B, contenant chacun un catalyseur différent. Un second mélange constitué par au moins des oléfines linéaires C₉-C₁₆, de préférence des oléfines linéaires C₁₀-C₁₄, accompagnées par des paraffines majoritairement C₁₀-C₁₄ est introduit par la conduite 5 directement dans le réacteur 6, le point d'injection étant situé entre les deux lits de catalyseurs A et B. A la sortie du réacteur 6, l'effluent est envoyé, par la conduite 7, vers l'échangeur de chaleur 3, puis, via la conduite 8, vers une première colonne de fractionnement 9. En tête de cette première colonne de fractionnement 9, la majorité du benzène en excès et n'ayant pas réagi est extrait et recyclé par la conduite 10. En fond de cette première colonne de fractionnement 9, on recueille une fraction qui est envoyée, par la conduite 11, vers une deuxième colonne de fractionnement 12. En tête de cette deuxième colonne de fractionnement 12, on recueille majoritairement, par la conduite 13, les oléfines linéaires C₉-C₁₆, de préférence C₁₀-C₁₄, non-transformées ainsi que les paraffines initialement présentes dans la charge. Au moins une partie de cet effluent peut être recyclée vers la conduite d'alimentation en benzène du réacteur 6. En fond de cette deuxième colonne de fractionnement 12, on soutire un mélange qui est envoyé, par la conduite 14, vers une troisième colonne de fractionnement 15. En tête de cette troisième colonne de fractionnement 15, on recueille majoritairement un mélange de 2-phénylalcane, 3-phénylalcane, 4-phénylalcane, 5-phénylalcane et 6-phénylalcane qui est envoyé au stockage par la conduite 16. En fond de cette troisième colonne de fractionnement 15, on soutire majoritairement, par la conduite 17, des dialkylbenzènes.

Les exemples 1 à 6 qui suivent ne limitent en rien la portée de l'invention. Ils sont donnés à titre illustratifs de manière à permettre à l'homme du métier de mieux comprendre la présente invention.

### Exemple 1 : préparation du catalyseur A à base de zéolithe Y

On utilise comme matière première une zéolithe NaY de formule NaAlO₂(SiO₂)_{2,5}. Cette zéolithe est soumise à 5 échanges successifs dans des solutions de nitrate d'ammonium de concentration 2M, à une température de 95°C, pendant une durée de 2 heures, et avec un rapport (volume de solution/poids de zéolithe) égal à 8 cm³/g. Le taux de sodium de la zéolithe NH₄Y obtenue est de 0,9% en poids. Ce produit est ensuite rapidement introduit dans un four préchauffé à 770°C et laissé pendant 4 heures en atmosphère statique. La zéolithe est ensuite soumise à un traitement acide dans les conditions suivantes : le rapport entre le volume d'acide nitrique 3N et le poids de solide est égal à 9 cm³/g, la température est de 95°C et la durée du traitement de 3 heures. Ensuite, un autre traitement dans les mêmes conditions est effectué, mais avec une solution d'acide nitrique 0,5N. La zéolithe ainsi obtenue possède une teneur pondérale en sodium de 0,1% et un rapport atomique Si/Al égal à 24. La zéolithe est mise en forme par extrusion avec de l'alumine (80% de zéolithe Y et 20% d'alumine). Les extrudés sont ensuite séchés puis calcinés à 550°C.

### Exemple 2 : préparation du catalyseur B à base de zéolithe mordénite (MOR)

On utilise comme matière première une zéolithe mordénite sous forme sodique dont la formule chimique à l'état anhydride est NaAlO₂(SiO₂)_{5,1} et sa teneur en sodium est de 5% en poids. On porte 100 grammes de cette poudre à reflux à 100°C pendant 2 heures dans une solution de nitrate d'ammonium de concentration 4M avec un rapport (volume de solution/poids de zéolithe) égal à 4 cm³/g. Cette opération d'échange cationique est répétée 3 fois. La teneur pondérale en sodium de la zéolithe NH₄MOR est d'environ 500 ppm (partie par million). La zéolithe est ensuite soumise à une attaque acide à l'aide d'une solution aqueuse d'acide nitrique 4,5 N : la zéolithe est portée à reflux dans cette solution aqueuse pendant 2 heures avec un rapport (volume de HNO₃/poids de zéolithe) égal à 4 cm³/g. Après ce traitement, la zéolithe est lavée à l'eau déminéralisée. La mordénite obtenue présente un rapport atomique Si/Al égal à 40 et une teneur en sodium égale à 20 ppm poids. Elle est ensuite mélangée avec un gel d'alumine (80% en poids de mordénite et 20% en poids de gel d'alumine). Le mélange obtenu est mis en forme sous forme d'extrudés de diamètre égal à environ 1,8 mm par passage au travers d'une filière. Les extrudés sont ensuite séchés dans une étuve à 120°C pendant une nuit, puis calcinés sous air sec à 550°C.

### Exemple 3 : alkylation du benzène par le dodécène-1 en présence du catalyseur A à base de zéolithe Y (non conforme à l'invention)

On utilise un réacteur contenant 50 cm³ de catalyseur A sous forme d'extrudés, préparé selon l'exemple 1.

Les conditions opératoires pour l'alkylation du benzène par le dodécène-1 sont les suivantes :
- température : 135°C
- pression : 4 MPa
- VVH = 1 h⁻¹ (cm³ charge (benzène+dodécène-1) / cm³ de catalyseur / heure)
- rapport molaire benzène/dodécène-1 : 5,5

On prépare une charge contenant 72% en poids de benzène et 28% en poids de dodécène-1.
Les résultats obtenus sont présentés dans le tableau 2.

**Tableau 2**

| Conversion du dodécène-1 (%) | 99,2 |
|---|---|
| Composition du produit obtenu (% poids) | |
| 2-phénylalcane | 26,2 |
| 3-phénylalcane | 21 |
| 4-phénylalcane | 18,9 |
| 5-phénylalcane | 11 |
| 6-phénylalcane | 10,9 |
| didodécylbenzène | 11 |
| résidu lourd | 1 |

La proportion de 2-phénylalcane dans le mélange constitué par le 2-phénylalcane, le 3-phénylalcane, le 4-, le 5-phénylalcane et le 6-phénylalcane est de 29,5%. Les produits monoalkylés représentent 88% en poids de la composition totale issue de la réaction d'alkylation réalisée au moyen d'un catalyseur à base de zéolithe Y.

### Exemple 4 : alkylation du benzène par le dodécène-1 en présence du catalyseur B à base de mordénite (non conforme à l'invention)

On utilise également un réacteur contenant 50 cm³ de catalyseur B sous forme d'extrudés tels que préparés dans l'exemple 2. On réalise le même essai que celui décrit dans l'exemple 3, dans les mêmes conditions opératoires et avec la même charge. Les résultats obtenus sont présentés dans le tableau 3 :

**Tableau 3**

| Conversion du dodécène-1 (%) | 98,7 |
|---|---|
| Composition du produit obtenu (% poids) | |
| 2-phénylalcane | 77.2 |
| 3-phénylalcane | 11 |
| 4-phénylalcane | 1,5 |
| 5-phénylalcane | 0,2 |
| 6-phénylalcane | 0 |
| | |
| didodécylbenzène | 9,6 |
| résidu lourd | 0,5 |

La proportion de 2-phénylalcane dans le mélange constitué par le 2-phénylalcane, le 3-phénylalcane, le 4-phénylalcane, le 5-phénylalcane et le 6-phénylalcane est de 85,9 %. Les produits monoalkylés représentent 89,9% en poids de la composition totale issue de la réaction d'alkylation réalisée au moyen d'un catalyseur à base de mordénite.

### Exemple 5 (conforme à l'invention) : Alkylation du benzène par le dodécène-1 dans deux réacteurs montés en série :

On utilise un dispositif comprenant deux réacteurs distincts. Conformément au procédé selon la présente invention, on utilise 30 cm³ de catalyseur A à base de zéolithe Y dans un premier lit au sein du premier réacteur, et 20 cm³ du catalyseur B à base de mordénite dans un deuxième lit au sein du second réacteur, la sélectivité en produits monoalkylés du catalyseur A étant inférieure à celle du catalyseur B. On prépare une charge contenant 84% poids de benzène et 16% poids de dodécène-1. Cette charge est injectée à l'entrée du premier réacteur contenant le catalyseur A. On injecte également du dodécène-1 pur entre les deux réacteurs. En entrée du premier réacteur, on injecte le benzène avec un débit de 34,5 cm³/h et le dodécène-1 avec un débit de 9,3 cm³/h. On injecte en entrée du second réacteur la totalité de l'effluent sortant du premier réacteur et du dodécène-1 à un débit de 6,2 cm³/h. Le premier réacteur fonctionne donc avec un rapport molaire benzène/dodécène-1 égal à 10,5.
Les conditions expérimentales sont les suivantes :
- température des 2 réacteurs : 135°C
- pression : 4 MPa
- VVH globale sur l'ensemble des 2 réacteurs : 1 h⁻¹ (exprimée en cm³ de charge (benzène+dodécène-1) / cm³ de catalyseur / heure)
Les résultats obtenus sont présentés dans le tableau 4. Les compositions indiquées sont celles déterminées en sortie du deuxième réacteur.

**Tableau 4**

| Conversion du dodécène-1 (%) | 99,1 |
|---|---|
| Composition du produit obtenu (% poids) | |
| 2-phénylalcane | 49,9 |
| 3-phénylalcane | 17,6 |
| 4-phénylalcane | 11,1 |
| 5-phénylalcane | 6,9 |
| 6-phénylalcane | 7 |
| | |
| didodécylbenzène | 7,1 |
| résidu lourd | 0,4 |

La proportion de 2-phénylalcane dans le mélange final constitué par le 2-phénylalcane, le 3-phénylalcane, le 4-phénylalcane, le 5-phénylalcane et le 6-phénylalcane est ici de 54%. Les produits monoalkylés représentent 92,5% en poids de la composition totale issue de la réaction d'alkylation.

On remarquera également que l'utilisation des deux catalyseurs permet d'augmenter de façon notable la sélectivité en monophénylalcanes (produits monoalkylés), en réduisant la quantité de composés di-alkylés et de composés lourds polyalkylés.

### Exemple 6 (comparatif): Alkylation du benzène par le dodécène-1 dans deux réacteurs montés en série :

On utilise une combinaison de deux catalyseurs : le catalyseur utilisé dans le premier lit est le catalyseur B à base de zéolithe mordénite décrit ci-avant, le catalyseur utilisé dans le second lit est un catalyseur à base de silice-alumine commercialisé par Condea sous la dénomination de Siralox 40. De manière à déterminer la sélectivité du catalyseur à base de silice-alumine en produits monoalkylés lorsqu'il est utilisé dans la réaction d'alkylation du benzène par le dodécène-1, on procède à un test similaire à celui décrit dans l'exemple 3 et dans les mêmes conditions opératoires. Après réaction, la conversion du dodécène-1 est égale à 100% et la proportion de produits monoalkylés dans le mélange constitué par les composés monoalkylés, dialkylés et résidu lourds est de 68,5%. La sélectivité en produits monoalkylés du catalyseur à base de silice-alumine est donc inférieure à celle du catalyseur B à base de mordénite utilisé dans le premier lit catalytique.

Pour la mise en oeuvre de la réaction d'alkylation du benzène par le dodécène-1 en présence des deux catalyseurs tels que précisés ci-dessus, on utilise un dispositif comprenant deux réacteurs distincts. On utilise 30 cm³ de catalyseur B à base de mordénite dans un premier lit au sein du premier réacteur, et 20 cm³ du catalyseur Siralox 40 (silice-alumine) dans un deuxième lit au sein du second réacteur.

On prépare une charge contenant 84% en poids de benzène et 16% en poids de dodécène-1. Cette charge est injectée à l'entrée du premier réacteur contenant le catalyseur B. On injecte également du dodécène-1 pur entre les deux réacteurs. On injecte en entrée du premier réacteur le benzène avec un débit de 34,5 cm³/h et le dodécène-1 avec un débit de 9,3 cm³/h. On injecte en entrée du second réacteur la totalité de l'effluent sortant du premier réacteur et du dodécène-1 à un débit de 6,2 cm³/h. Le premier réacteur fonctionne donc avec un rapport molaire benzène/dodécène-1 égal à 10,5.
Les conditions expérimentales sont les suivantes :
- température des 2 réacteurs : 135°C
- pression : 4 MPa
- VVH globale sur l'ensemble des 2 réacteurs : 1 h⁻¹ (exprimée en cm³ de charge (benzène+dodécène-1) / cm³ de catalyseur / heure

Les résultats obtenus sont présentés dans le tableau 5. Les compositions indiquées sont celles déterminées en sortie du deuxième réacteur.

**Tableau 5**

| Conversion du dodécène-1 (%) | 100 |
|---|---|
| Composition du produit obtenu (% poids) | |
| 2-phénylalcane | 46,3 |
| 3-phénylalcane | 15,2 |
| 4-phénylalcane | 10,0 |
| 5-phénylalcane | 6,2 |
| 6-phénylalcane | 3,5 |
| | |
| didodécylbenzène | 15,6 |
| résidu lourd | 3,2 |

La proportion de 2-phénylalcane dans le mélange final constitué par le 2-phénylalcane, le 3-phénylalcane, le 4-phénylalcane, le 5-phénylalcane et le 6-phénylalcane est ici de 57%. Les produits monoalkylés représentent 81,2% en poids de la composition totale issue de la réaction d'alkylation.

La combinaison de deux catalyseurs disposés de telle manière que la sélectivité en produits monoalkylés du catalyseur occupant le premier réacteur soit supérieure à celle du catalyseur occupant le second réacteur permet d'ajuster la teneur en 2-phénylalcane à une valeur voisine de celle obtenue par la combinaison des deux catalyseurs conformément au procédé selon l'invention i.e., disposés de telle manière que la sélectivité en produits monoalkylés du catalyseur occupant le premier réacteur soit inférieure à celle du catalyseur occupant le second réacteur. En revanche, le procédé selon l'invention conduit à une amélioration sensible du rendement en produits monoalkylés (92,5% en poids contre 81,2% en poids) au détriment de la production de composés di-alkylés et de composés lourds polyalkylés (7,5% contre18,8% dans l'exemple comparatif).

## Revendications

1. Procédé de production de phénylalcanes comprenant une réaction d'alkylation d'au moins un composé aromatique par au moins une oléfine linéaire ayant de 9 à 16 atomes de carbone par molécule réalisée en présence d'au moins deux catalyseurs zéolithiques différents et utilisés dans au moins deux zones réactionnelles distinctes, la sélectivité en produits monoalkylés du catalyseur zéolithique contenu dans la première zone réactionnelle étant inférieure à celle du catalyseur zéolithique contenu dans la deuxième zone réactionnelle, située en aval de la première dans le sens de circulation des fluides.

2. Procédé de production de phénylalcanes selon la revendication 1 tel que la zéolithe présente dans chacun desdits catalyseurs zéolithiques est choisie dans le groupe constitué par les zéolithes appartenant aux types structuraux FAU, MOR, MTW, OFF, MAZ, BEA et EUO.

3. Procédé de production de phénylalcanes selon la revendication 2 tel que la zéolithe est une zéolithe de type structural FAU.

4. Procédé de production de phénylalcanes selon la revendication 3 tel que la zéolithe est une zéolithe Y.

5. Procédé de production de phénylalcanes selon la revendication 2 tel que la zéolithe est une zéolithe de type structural MOR.

6. Procédé de production de phénylalcanes selon la revendication 5 tel que la zéolithe est une zéolithe mordénite.

7. Procédé de production de phénylalcanes selon l'une des revendications 1 à 6 tel que les zéolithes contenues dans chacun des catalyseurs zéolithiques diffèrent l'une de l'autre par leur type structural et/ou par la composition chimique de leur charpente cristalline.

8. Procédé de production de phénylalcanes selon l'une des revendication 1 à 7 tel que le catalyseur zéolithique de la première zone réactionnelle contient une zéolithe Y et le catalyseur zéolithique de la deuxième zone réactionnelle contient une zéolithe mordénite.

9. Procédé de production de phénylalcanes selon l'une des revendications 1 à 8 tel que l'oléfine est mélangée avec le composé aromatique en amont de la première zone réactionnelle.

10. Procédé de production de phénylalcanes selon l'une des revendications 1 à 8 tel qu'une première fraction de l'oléfine est mélangée avec le composé aromatique en amont de la première zone catalytique et tel qu'une deuxième fraction de l'oléfine est mélangée avec au moins une partie des effluents issus de la première zone réactionnelle.

11. Procédé de production de phénylalcanes selon la revendication 10 tel que la quantité d'oléfine contenue dans ladite première fraction est telle que sensiblement la totalité de l'oléfine est consommée dans la première zone réactionnelle.

12. Procédé de production de phénylalcanes selon l'une des revendications 1 à 11 tel que ladite réaction d'alkylation est suivie d'au moins une étape de séparation des réactifs en excès.

13. Procédé de production de phénylalcanes selon l'une des revendications 1 à 12 tel que ladite réaction d'alkylation est suivie d'au moins une étape de séparation des composés monoalkylés issus de la réaction.

14. Procédé de production de phénylalcanes selon l'une des revendications 1 à 13 dans lequel l'oléfine linéaire est une oléfine ayant de 10 à 14 atomes de carbone par molécule.

15. Procédé de production de phénylalcanes selon l'une des revendications 1 à 14 dans lequel l'oléfine linéaire est le dodécène-1.

16. Utilisation du procédé selon l'une des revendications 1 à 15 pour la fabrication de détergents.

## Claims

1. A process for producing phenylalkanes comprising alkylating at least one aromatic compound by at least one linear olefin containing 9 to 16 carbon atoms per molecule carried out in the presence of at least two different catalysts and used in at least two distinct reaction zones, the selectivity for monoalkylated products of the zeolitic catalyst contained in the first reaction zone being lower than that of the zeolitic catalyst contained in the second reaction zone located downstream of the first reaction zone in the direction of fluid movement.

2. A process for producing phenylalkanes according to claim 1, in which the zeolite present in each of said zeolitic catalysts is selected from the group formed by zeolites with structure types FAU, MOR, MTW, OFF, MAZ, BEA and EUO.

3. A process for producing phenylalkanes according to claim 2, in which the zeolite is a zeolite with structure type FAU.

4. A process for producing phenylalkanes according to claim 3, in which the zeolite is a Y zeolite.

5. A process for producing phenylalkanes according to claim 2, in which the zeolite is a zeolite with structure type MOR.

6. A process for producing phenylalkanes according to claim 5, in which the zeolite is a mordenite zeolite.

7. A process for producing phenylalkanes according to one of claims 1 to 6, in which the zeolites contained in each of the different zeolitic catalysts differ from each other in their structure type and/or in the chemical composition of their crystalline framework.

8. A process for producing phenylalkanes according to one of claims 1 to 7, in which the zeolitic catalyst of the first reaction zone contains a Y zeolite and the zeolitic catalyst in the second reaction zone contains a mordenite zeolite.

9. A process for producing phenylalkanes according to one of claims 1 to 8, in which the olefin is mixed with the aromatic compound upstream of the first reaction zone.

10. A process for producing phenylalkanes according to one of claims 1 to 8, in which a first fraction of the olefin is mixed with the aromatic compound upstream of the first catalytic zone and in which a second fraction of the olefin is mixed with at least a portion of the effluents from the first reaction zone.

11. A process for producing phenylalkanes according to claim 10, in which the quantity of olefin contained in said first fraction is such that substantially all of the olefin is consumed in the first reaction zone.

12. A process for producing phenylalkanes according to one of claims 1 to 11, in which said alkylation reaction is followed by at least one step for separating the excess reactants.

13. A process for producing phenylalkanes according to one of claims 1 to 12, in which said alkylation reaction is followed by at least one step for separating monoalkylated compounds deriving from the reaction.

14. A process for producing phenylalkanes according to one of claims 1 to 13, in which the linear olefin is an olefin containing 10 to 14 carbon atoms per molecule.

15. A process for producing phenylalkanes according to one of claims 1 to 14, in which the linear olefin is 1-dodecene.

16. Use of a process according to one of claims 1 to 15, for the production of detergents.

## Patentansprüche

1. Verfahren zur Herstellung von Phenylalkanen, das eine Alkylierungsreaktion mindestens einer aromatischen Verbindung durch mindestens ein lineares Olefin umfasst, das 9 bis 16 Kohlenstoffatome je Molekül aufweist, die in Gegenwart von mindestens zwei verschiedenen Katalysatoren ausgeführt wird, und die in mindestens zwei unterschiedlichen Reaktionszonen verwendet werden, wobei die Selektivität für monoalkylierte Produkte des zeolitischen Katalysators, der in der ersten Reaktionszone enthalten ist, geringer ist als jene des zeolithischen Katalysators, der in der zweiten Reaktionszone enthalten ist, der stromabwärts des ersten in Zirkulationsrichtung der Fluide angeordnet ist.

2. Verfahren zur Herstellung von Phenylalkanen nach Anspruch 1, so dass der Zeolith, der in jedem der zeolithischen Katalysatoren vorhanden ist, ausgewählt ist aus der Gruppe, bestehend aus den Zeolithen, die zu den Strukturtypen FAU, MOR, MTW, OFF, MAZ, BEA und EUO gehören.

3. Verfahren zur Herstellung von Phenylalkanen nach Anspruch 2, so dass der Zeolith ein Zeolith mit dem Strukturtyp FAU ist.

4. Verfahren zur Herstellung von Phenylalkanen nach Anspruch 3, so dass der Zeolith ein Y-Zeolith ist.

5. Verfahren zur Herstellung von Phenylalkanen nach Anspruch 2, so dass der Zeolith ein Zeolith mit dem Strukturtyp MOR ist.

6. Verfahren zur Herstellung von Phenylalkanen nach Anspruch 5, so dass der Zeolith ein Mordenit-Zeolith ist.

7. Verfahren zur Herstellung von Phenylalkanen nach einem der Ansprüche 1 bis 6, so dass die Zeolithen, die in jedem der zeolithischen Katalysatoren enthalten sind, sich durch ihren Strukturtyp und/oder durch die chemische Zusammensetzung ihres Kristallgerüsts voneinander unterscheiden.

8. Verfahren zur Herstellung von Phenylalkanen nach einem der Ansprüche 1 bis 7, so dass der zeolithische Katalysator der ersten Reaktionszone einen Y-Zeolithen enthält, und der zeolithische Katalysator der zweiten Reaktionszone einen Mordenit-Zeolithen enthält.

9. Verfahren zur Herstellung von Phenylalkanen nach einem der Ansprüche 1 bis 8, so dass das Olefin mit der aromatischen Verbindung stromaufwärts der ersten Reaktionszone gemischt wird.

10. Verfahren zur Herstellung von Phenylalkanen nach einem der Ansprüche 1 bis 8, so dass eine erste Fraktion des Olefins mit der aromatischen Verbindung stromaufwärts der ersten katalytischen Zone gemischt wird, und dass eine zweite Fraktion des Olefins mit mindestens einem Teil der Abflüsse gemischt wird, die aus der ersten Reaktionszone stammen.

11. Verfahren zur Herstellung von Phenylalkanen nach Anspruch 10, so dass die Menge an Olefin, die in der ersten Fraktion enthalten ist, so ist, dass im Wesentlichen das gesamte Olefin in der ersten Reaktionszone verbraucht wird.

12. Verfahren zur Herstellung von Phenylalkanen nach einem der Ansprüche 1 bis 11, so dass auf die Alkylierungsreaktion mindestens ein Schritt der Abscheidung der überschüssigen Reagenzien folgt.

13. Verfahren zur Herstellung von Phenylalkanen nach einem der Ansprüche 1 bis 12, so dass auf die Alkylierungsreaktion mindestens ein Schritt der Abscheidung der monoalkylierten Verbindungen, die aus der Reaktion stammen, folgt.

14. Verfahren zur Herstellung von Phenylalkanen nach einem der Ansprüche 1 bis 13, wobei das lineare Olefin ein Olefin ist, das 10 bis 14 Kohlenstoffatome je Molekül aufweist.

15. Verfahren zur Herstellung von Phenylalkanen nach einem der Ansprüche 1 bis 14, wobei das lineare Olefin 1-Dodecen ist.

16. Verwendung des Verfahrens gemäß einem der Ansprüche 1 bis 15 zur Fertigung von Detergenzien.
